# EUROPEAN PATENT SPECIFICATION

(11) **EP 4 186 449 B1**
(45) Date of publication and mention of the grant of the patent: **24.07.2024**
(21) Application number: 21210491.3
(22) Date of filing: 25.11.2021
(51) Int. Cl.: A61B 17/34

(54) **A TROCAR FIXATION ASSEMBLY**
TROKARFIXIERUNGSANORDNUNG
ENSEMBLE DE FIXATION DE TROCART

(43) Date of publication of application: 31.05.2023
(73) Proprietor: Mölnlycke Health Care AB, 402 52 Göteborg (SE)
(72) Inventor: ROSENGREN, Oscar, Hovås (SE); APELSTEDT, Kristoffer, Jönköping (SE)
(74) Representative: Kransell & Wennborg KB

(56) References cited:
- EP-A1- 3 210 553
- US-A- 5 267 970
- US-A- 5 792 112
- US-A1- 2010 057 010
- US-A1- 2021 068 917

## Description

### TECHNICAL FIELD

The present disclosure generally relates to a trocar fixation assembly comprising a fixation device and a medical dressing. The present disclosure also relates to a trocar system comprising the trocar fixation assembly.

### BACKGROUND

Minimal invasive surgery (MIS) is a technique for performing surgery that is associated with less pain, shorter recovery and fewer complications compared to traditional open surgery.

In an MIS procedure, a small incision is typically made through the skin of a patient, and a trocar is thereafter inserted into the incision. The trocar serves as a working channel that allows for the entry of various surgical instruments and cameras into a body cavity, e.g. an abdominal cavity.

A trocar typically comprises a cannula and an obturator. The cannula is a hollow and tube-shaped plastic or metallic shaft that is placed inside the patient to provide access to the body cavity during the MIS procedure. The obturator is a sharp tool that allows the cannula to penetrate the body cavity for initial placement. When the cannula has entered the body cavity, the obturator is removed.

To date, the commonly used trocars in MIS procedures include balloon trocars, and Hasson trocars, respectively. A Hasson trocar is a non-bladed trocar that stays in place by means of sutures anchored to the abdominal wall fascia. A balloon trocar comprises a balloon at the end of the trocar that is inflated with air. Instead of utilizing sutures for fixing the trocar, the inflated balloon anchors the trocar in the abdomen.

Fixating and stabilizing the trocar, particularly the cannula, is typically challenging during most MIS procedures. Both the balloon and Hasson trocar system suffer from disadvantages with respect to fixation and stabilization. For example, the inflated balloon of a balloon trocar may rupture, which is associated with risks for the patient. Furthermore, a balloon trocar cannot simply be removed from the body cavity since the balloon must first be deflated.

The Hasson trocar is cumbersome in the sense that fixation and stabilization require the aid of sutures to fixate the trocar in the body cavity. Upon removal of the trocar, the sutures must first be removed. Furthermore, the cannula must typically comprise ridges to fix the skin mechanically.

Trocar cannulas may either have a smooth, threaded, or ribbed outer surface. Threaded and ribbed cannulas provide for some degree of retention, which may prevent the cannula from moving in and out of the body cavity. However, cannulas with a smooth outer surface are easily dislocated and may project out of the body cavity or deeper into the body cavity during use. Such dislocation is risky during surgery, and should be avoided.

Another challenge during MIS procedures is sterility and keeping the surgical site and the area circumventing the trocar clean and free from blood.

US 2010/057010 A1 discloses a fixation device for holding a medical instrument such as a trocar, on the skin of a patient. US 2021/068917 A1 discloses a medical device stabilizer having a base plate and a compressible medical device grip in the form of a resilient ball with a guide bore on the base plate which can articulate for gripping and positioning medical devices. US 5267970 A discloses a device for anchoring a trocar sleeve to a patient's body.

Accordingly, there is room for improvements in fixating and stabilizing the trocar in a sterile, simplified and less cumbersome manner. Such fixation and stabilization means should prevent dislocation of the trocar cannula during use and facilitate initial attachment, and removal of the trocar from the body cavity.

### SUMMARY

In view of the above-mentioned problems, it is an object of the present disclosure to provide improvements with respect relieving the burden for surgical staff and the patients and providing a facilitated and improved means to handle and fixate a trocar during minimal invasive surgery. The invention is defined by independent claim 1, while further embodiments are defined by the dependent claims.

According to a first aspect, there is provided a trocar fixation assembly comprising a fixation device and a medical dressing, wherein the fixation device comprises an annular member defining a channel for receiving a trocar; the annular member being disposed about a longitudinal center line, wherein the fixation device is movable between a first configuration, which allows for a trocar to be moved along the longitudinal center line of the annular member, and a second configuration, which allows for a trocar to be fixed in a locked position, wherein in the second configuration, the cross-sectional area of at least one portion of the channel is smaller than the cross-sectional area of the at least one portion of the channel in the first configuration.

The present disclosure is based on the realization that a trocar fixation assembly according to the present disclosure greatly facilitates the minimally invasive surgical procedure for the surgeon and for the surgical staff. The trocar fixation assembly provides for a safe surgical procedure, avoiding undesired intervention in the patient during fixation and stabilization of the trocar.

The trocar fixation assembly is configured to be shifted between a first and a second configuration. In the first configuration, the cross-sectional area of at least a portion of the channel is larger, and a trocar; i.e. trocar cannula may be moved along the longitudinal center line; i.e. into and out of the body cavity. Accordingly, in this configuration, the surgeon or surgical staff may insert the trocar into the body cavity and adjust the depth of the cannula such that it is inserted in a desired position.

In the second configuration, the cross-sectional area of the portion of the channel is smaller and the trocar cannula is fixed in position. Accordingly, the trocar cannula is prevented from moving in the longitudinal direction (and in any direction).

The trocar fixation assembly allows for a trocar cannula to be fixed in a position very close to the skin surface, which is beneficial during e.g. hernia repair surgery.

Since no part of the trocar fixation assembly remains inside the body (as is the case with balloon trocars) or requires fixation by means of sutures to the skin (as is the case with Hasson trocars), the trocar fixation assembly provides a safe and simplified means to fixate a trocar in position during minimal invasive surgery. Furthermore, the attachment and detachment of the trocar fixation assembly is significantly facilitated.

The trocar is prevented from moving into and out of the body cavity during surgery. Such movement is undesired since moving the trocar too deep into the body cavity may be harmful to the patient. Furthermore, if the trocar slips out of the body cavity during surgery, this may result in failure of the MIS procedure.

Even though the trocar fixation assembly of the present disclosure is not limited to a particular trocar or a particular cannula, the trocar fixation assembly is particularly beneficial for trocars with cannulas having a smooth outer surface. Such cannulas have an enhanced tendency to slide into and out of the body cavity during surgery.

In exemplary embodiments, the second configuration is a relaxed configuration and wherein the first configuration is a retracted configuration.

The relaxed configuration may also be referred to as the "closed" or "locked" configuration, in which the trocar is fixed in position.

The retracted configuration may be referred to as the "open" configuration, in which the trocar is movable in the longitudinal direction.

The shifting from the second, relaxed configuration to the first, retracted configuration typically requires a force, e.g. a pushing or a clamping force.

In exemplary embodiments, the fixation device comprises locking means configured to move the fixation device between the first and the second configurations.

In exemplary embodiments, the locking means comprises a spring member.

The annular member comprises interior walls and exterior walls, wherein the spring member is a spring wire encircling at least a portion of the interior walls of the annual member.

In the second, relaxed configuration, at least a portion of the spring wire projects radially from the interior walls into the channel. Accordingly, a trocar cannula is prevented from being inserted into the channel. However, once inserted, the spring wire secures that the trocar is fixed in position by a clamping mechanism. Hence, in the portion of the channel where the spring wire is arranged, the cross-sectional area is smaller than the cross-sectional area of the remaining portions of the channel.

In the first, retracted configuration, the spring wire is retracted against the interior walls of the annular member. Accordingly, the cross-sectional area of the portion of the channel where the spring wire is arranged may correspond to the cross-sectional area of the remaining portions of the channel.

It is, however, conceivable that the spring wire is wound around the entire interior walls of the annular member. Nevertheless, the cross-sectional area of at least one portion of the channel in the second, relaxed configuration will be smaller than the cross-sectional area in the same portion of the channel in the first configuration.

The spring wire may be operably engaged with at least a first locking tab, wherein the first locking tab projects radially from the exterior walls of the annular member.

The surgeon may easily grasp the first locking tab during insertion and positioning of the trocar at a surgical incision.

The first locking tab may be movable between a first position and a second position, wherein the movement of the first locking tab from the first to the second position causes the spring wire to retract such that the fixation device is moved from the second to the first configuration

The movement of the first locking tab between a first and a second position causes the spring wire to retract such that the fixation device adopts the first, open configuration. After the trocar has been fixed in position, the surgeon may simply release the tab, such that that trocar is "clamped" in the fixed position by means of the spring member

In exemplary embodiments, a distal end of the spring wire spring is connected to or forms part of the first locking tab.

The spring wire typically has two distal ends. A first distal end may be connected to or form part of the first locking tab. The second distal end may be positioned against the interior walls and may be utilized to anchor the spring wire to the interior walls of the annular member.

For example, the annular member may comprise a slot to connect the first distal end of the spring wire to the first locking tab projecting from the exterior walls of the channel.

In exemplary embodiments, the annular member comprises a second locking tab projecting radially from the exterior walls of the annular member, wherein the first locking tab is moveable in a circumferential direction with respect to the second locking tab.

The second locking tab facilitates the shifting between the first and the second positions. The operator may grasp the first and the second tabs with his/her fingers and by moving the first locking tab towards the second locking tab in a circumferential direction, i.e. along part of the circumference of the annular member, the spring member, e.g. spring wire retracts.

In exemplary embodiments, the medical dressing comprises a centrally disposed aperture configured to encircle the annular member.

The medical dressing typically encircles the annular member such that no gaps are formed between the medical dressing and the fixation device. This is to prevent potential entry of contaminants. The parts of the medical dressing surrounding the annular member of the fixation device may be adhesively attached to the skin of a patient.

In exemplary embodiments, the medical dressing extends uninterrupted around the annular member.

A tight seal is thereby provided, and the sterility is improved in the area circumventing the surgical site.

In exemplary embodiments, the medical dressing comprises a backing layer and an adhesive skin contact layer.

The adhesive skin contact layer secures a tight fit to the skin, and the dressing is fixed in place in the area circumventing the surgical site. Blood and body fluids exuded from the surgical site may be evaporated through the backing layer.

In exemplary embodiments, the adhesive skin contact layer comprises a silicone based adhesive.

A silicone based adhesive is skin-friendly and gentle to the skin. The silicone based adhesive allows for the dressing to be removed from the skin in a gentle manner without causing trauma.

In exemplary embodiments, the medical dressing comprises an absorbent pad between the backing layer and the adhesive skin contact layer.

An absorbent pad is beneficial to absorb the blood resulting from the surgical intervention. It is desirable to prevent blood from accumulating at the skin, and instead secure removal and proper handling of the blood by means of the absorbent pad. Accordingly, contaminating microorganisms are prevented from accumulating at the skin, and the sterility is improved.

In exemplary embodiments, the pad comprises a polyurethane foam.

A polyurethane foam is beneficial since it is capable of absorbing large amounts of fluids, and also has a pressure relieving effect. The pressure relieving effect is beneficial to prevent pressure ulcers from occurring. A dressing comprising a polyurethane foam is also conformable.

According to another aspect, there is provided a trocar system comprising a trocar fixation assembly and a trocar

In exemplary embodiments, the trocar comprises a cannula, wherein the cannula comprises an interior surface and an exterior surface, wherein the exterior surface of the cannula is smooth.

The trocar fixation assembly of the present disclosure is particularly beneficial for trocar cannulas having a smooth surface since these have an enhanced tendency to dislocate into and out of the body cavity during surgery.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 illustrates a trocar fixation assembly according to an exemplary embodiment of the present disclosure applied to the skin of a patient.
Figure 2A illustrates trocar fixation assembly according to an exemplary embodiment of the present disclosure, including a zoomed-in, cross-sectional view of the medical dressing.
Figure 2B illustrates a split view of the trocar fixation assembly in figure 2A.
Figure 3A illustrates a trocar fixation assembly according to an exemplary embodiment of the present disclosure in the second, relaxed configuration.
Figure 3B illustrates a cross-sectional view of the trocar fixation assembly along the line A-A in figure 3A.
Figure 4A illustrates a trocar fixation assembly according to an exemplary embodiment of the present disclosure in the first, retracted configuration.
Figure 4B illustrates a cross-sectional view of the trocar fixation assembly along the line A-A in figure 4A.
Figure 5A illustrates the insertion of a trocar in the trocar fixation assembly according to an exemplary embodiment of the present disclosure.
Figure 5B illustrates a cross-sectional view of the trocar fixation assembly along the line A-A in figure 5A.
Figure 6A illustrates a trocar fixation assembly according to an exemplary embodiment of the present disclosure in conjunction with a trocar.
Figure 6B illustrates a cross-sectional view of the trocar fixation assembly along the line A-A in figure 6A.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

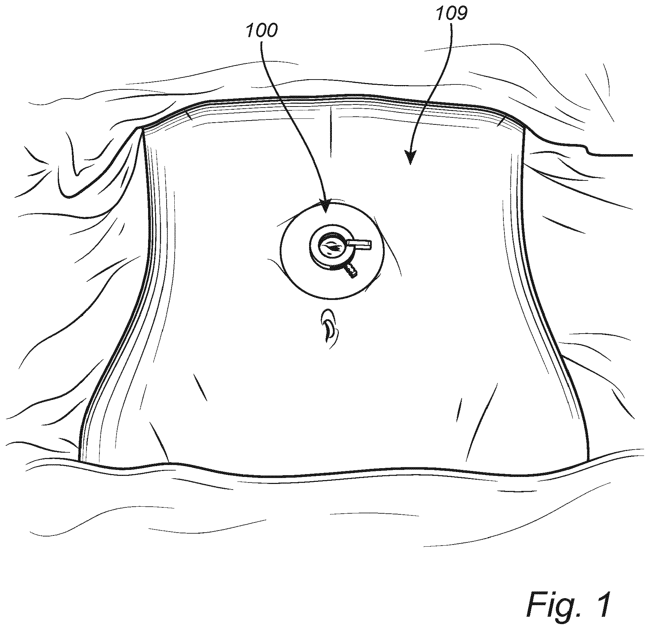

With reference to figure 1, a trocar fixation assembly 100 according to an exemplary embodiment of the present disclosure is schematically illustrated.

The trocar fixation assembly has been attached to the skin 109 of a patient. An incision allowing for the entry of a trocar may be made before or after the trocar fixation assembly has been attached to the skin 109.

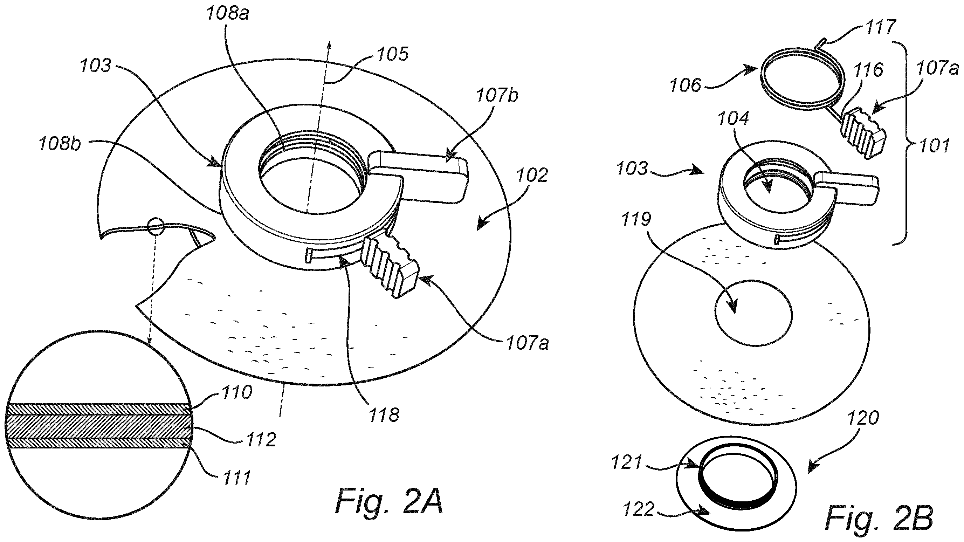

With reference to figures 2A and 2B, the trocar fixation assembly 100 comprises a fixation device 101 and a medical dressing 102, wherein the fixation device 101 comprises an annular member 103 defining a channel 104 for receiving a trocar; the annular member being disposed about a longitudinal center line 105, wherein the fixation device 101 is movable between a first configuration, which allows for a trocar to be moved along the longitudinal center line 105 of the annular member 103, and a second configuration, which allows for a trocar to be fixed in a locked position, wherein in the second configuration, the cross-sectional area of at least one portion of the channel 104 is smaller than the cross-sectional area of the at least one portion of the channel 104 in the first configuration.

As used herein, the term "trocar fixation assembly" means an assembly comprising at least a fixation device and a medical dressing. The medical dressing may be fixedly or detachably attached to the fixation device. The medical dressing is typically arranged to extend around the contour of the fixation device and to be arranged in contact with the skin of the patient. The trocar fixation assembly is to be used in conjunction with a trocar. More particularly, the trocar fixation assembly is used to receive and engage with a trocar cannula.

As used herein, the term "trocar" means a device through which a minimal invasive surgery can be accomplished. The trocar typically comprises at least a cannula; i.e. a hollow tube shaped member, and a gas-tight valve. The trocar allows for the entry of surgical instruments, such as graspers, scissors, staplers, cameras etc.

The "annular member" is an annular; i.e. ring-shaped element defining a channel. The channel defined by the annular member has a circular cross-section. The first annular member typically extends uninterrupted around the channel. Accordingly, the first annular member forms a closed path around the channel and is not openable. The annular member may be arranged in contact with the skin of a patient. Alternatively, it may be connected to a skin-attachment element that is arranged in contact with the skin. The annular member is not limited to a particular material but may be formed by any skin compatible or skin friendly material. For example, the first annular member may be formed by a plastic material.

In the second configuration of the fixation device, the cross-sectional area of at least one portion of the channel may be from 5 to 20%, e.g. from 10-15% smaller than the cross-sectional area of that portion of the channel in the first configuration

The second configuration of the fixation device may be a relaxed configuration and wherein the first configuration may be a retracted configuration.

As used herein, the term "relaxed configuration" means the configuration that the fixation device has in the absence of a force exerted on the fixing device, e.g. a pushing, clamping or retraction force. The relaxed configuration may also be referred to as a "closed" or "locked" configuration. This is the configuration that the trocar fixation assembly has prior to insertion of a trocar.

As used herein, the term "retracted configuration" means the configuration has that the fixation device has when subjected to a force, e.g. a pushing, clamping or retraction force. The retracted configuration may also be referred to as an "open" configuration.

The trocar fixation assembly 100 of the present disclosure allows for a trocar to be fixed in a safe and skin friendly manner compared to e.g. conventional Hasson or balloon trocars. No parts of the trocar fixation assembly reside inside the body cavity (e.g. no sutures or balloon), and the attachment and detachment of the trocar fixation assembly is significantly facilitated.

The second, relaxed configuration also allows for a trocar cannula to be fixed at a minimal distance from the skin, which may be desirable during e.g. hernia repair surgery.

The fixation device 101 may comprise locking means configured to move the fixation device between the first and the second configurations.

Typically, the locking means comprises a spring member 106.

As best illustrated in figure 2A, the annular member 103 comprises interior walls 108a and exterior walls 108b, wherein the spring member is a spring wire encircling at least a portion of the interior walls 108a of the annular member 103.

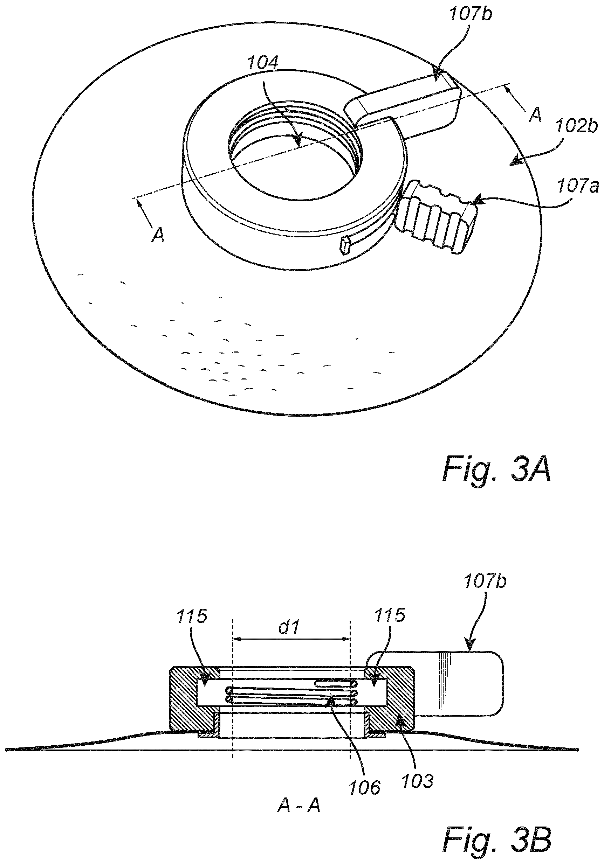

As illustrated in figure 3B, in the second configuration, at least a portion of the spring wire 106 projects radially into the channel 104 of the annular member 103. This is illustrated by the distance, d1 in figure 3B. In this configuration, the cross-sectional area of at least one portion of the channel 104 (i.e. where the spring wire is arranged) is smaller than the cross-sectional area of same portion of the channel 104 in the first configuration. The radially projecting portion of the spring wire 106 prevents a trocar from being inserted into the channel 104. It also secures a clamping mechanism towards a trocar cannula inserted into the channel 104.

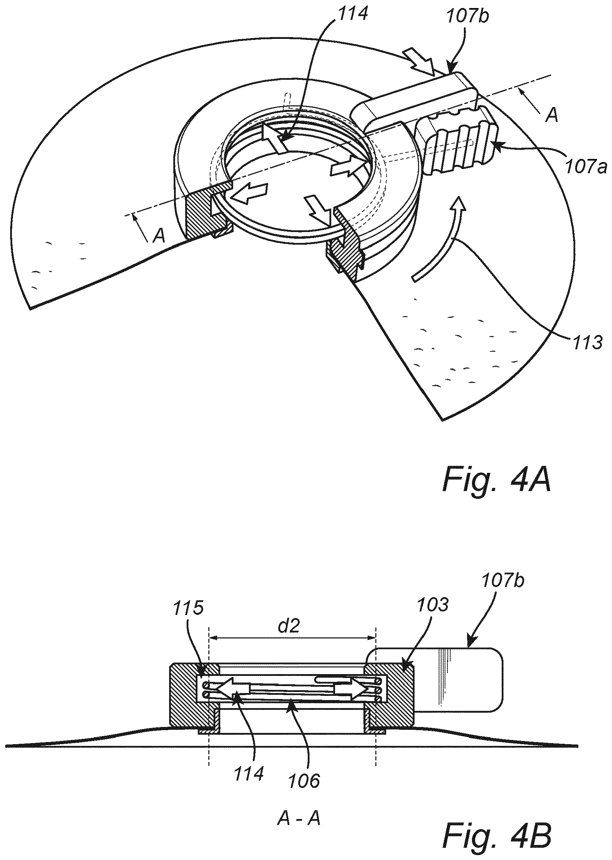

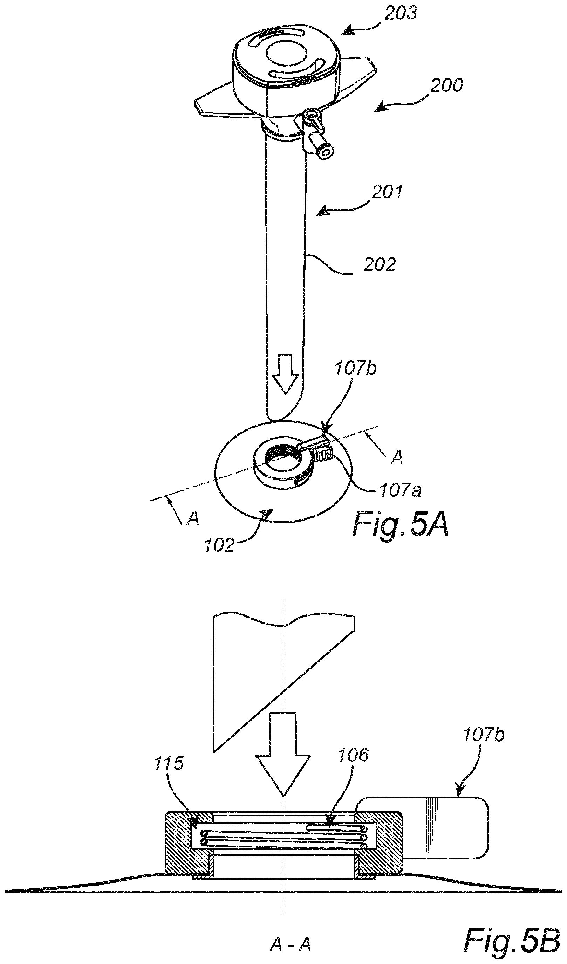

As illustrated in figures 4A and 4B, where the first configuration has been shifted to the second configuration, the spring wire 106 is retracted. Accordingly, the at least one radially projecting portion of the spring wire 106 retracts against the interior walls 108a of the annular member 103. Accordingly, the cross-sectional area of the portion of the channel 104, where the spring wire 106 is arranged increases. This is illustrated by the distance, d2, in figure 4B. Accordingly, a trocar may be inserted into the channel 104, as illustrated in figures 5A and 5B.

The spring wire 106 may be operably engaged wit at least a first locking tab 107b. As illustrated in e.g. figure 2A, the first locking tab 107a projects radially from the exterior walls 108b of the annular member 103.

As best illustrated in figure 2B, a distal end 116 of the spring wire 106 is connected to or forms part of the first locking tab 107a.

The distal end 116 of the spring wire connected to or forming part of the first locking tab 107a may be referred to as a "first distal end".

The second distal end 117 of the spring wire may be utilized to anchor the spring wire 106 at the interior walls 108a of the annular member 103. As illustrated in figure 2B, the second distal end 117 is bent at an angle such that it can be anchored to the interior walls 108a of the annular member 103.

In the area where the spring wire is arranged, the interior walls 108a may comprise one or more annular indentations (see 115 in e.g. figure 3B). The annular indentations 115 allow for the spring wire 106 to retract such that the cross-sectional area of the channel 104 is increased, and such that the first, open configuration can be adopted.

The spring wire may be wound at least one lap along the perimeter of the interior walls 108a of the annular member 103. It is also conceivable that the spring wire is wound a plurality of laps along the perimeter of the interior walls 108a of the annular member 103. The spring member may, in alternative embodiments be a spring coil. Accordingly, the spring member is wound around the entire interior walls 108a of the annular member 103.

The first locking tab 107a may be easily accessed and grasped by an operator.

As best illustrated in figure 4A, the first locking tab 107a is movable between a first position and a second position (indicated by the arrow 113), wherein the movement of the first locking tab 107a from the first to the second position causes the spring member to retract such that the fixation device 101 is moved from the second to the first configuration. The retraction of the spring member 106 is illustrated by the arrows 114 in figures 4A and 4B.

With reference to e.g. figure 2A, the annular member 103 may comprise a slot 118 extending through the exterior walls of the annular member 103. The slot 118 has a circumferential extension. The slot 118 allows for the spring wire 106 to be operably engaged with the first locking tab 107a projecting outwardly from the exterior walls 108b of the annular member 103.

Furthermore, the circumferentially extending slot 118 allows for the spring member to move between the first and the second positions.

Accordingly, the first locking tab 107a may be movable in a circumferential direction of the annular member 103 (see arrow 113 in figure 4A).

In embodiments, the annular member 103 comprises a second locking tab 107b projecting radially from the exterior walls 108b of the annular member 103, wherein the first locking tab 107a is moveable in a circumferential direction with respect to the second locking tab 107b. Accordingly, the firs locking tab 107a may be moved along a portion of the circumference of the annular member 103.

The second locking tab 107b may or may not be movable.

In the embodiment illustrated in the figures, the second locking tab 107b is a fixed tab. Accordingly, it is not moveable in any direction. Instead, the second locking tab 107b serves as a support to facilitate the movement of the first locking tab between the first and the second positions in order to facilitate shifting between the first, and the second configurations of the fixation member 101.

The second locking tab 107b projects radially from the exterior surface of the annular member 103. The operator may easily grasp the first 107a and second 107b locking tabs by his/her thumb and forefinger to shift between the first, and the second configurations of the fixation device 101.

As illustrated in figures 5A and 5B, the first locking tab 107a has been moved towards the second locking tab 107b enabling the insertion of a trocar 200 along the longitudinal center line 105 of the annular member 103.

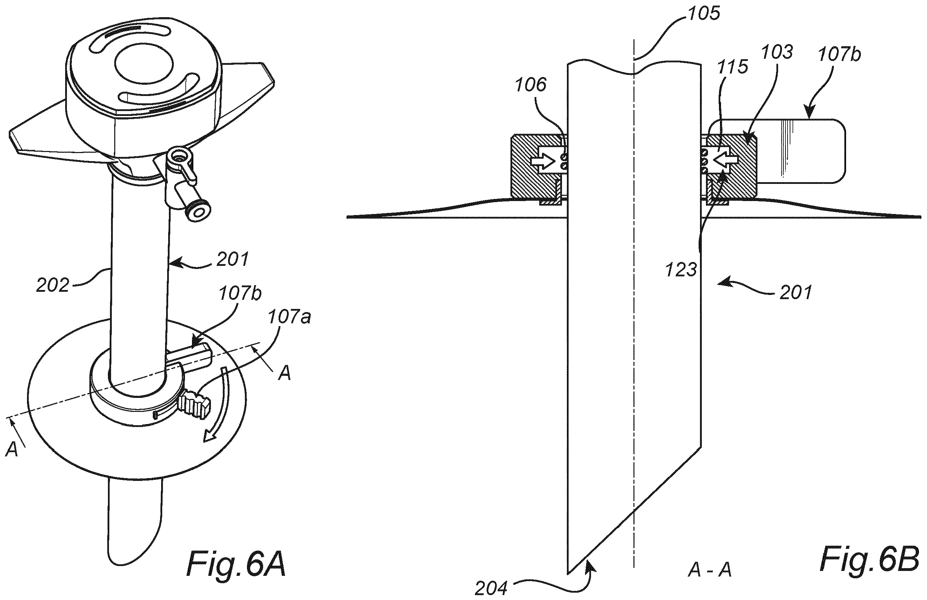

Figures 6A and 6B illustrate the trocar fixation assembly when a trocar 200 has been inserted into the channel 104 of the annular member 103. When the trocar 200 has been inserted, the operator may release the first 107a and/or second 107b tab. The spring wire clamps the trocar cannula 201 such that it becomes completely fixed (indicated by the arrows 123 in figure 6B).

The channel 104 of the annular member has a circular cross-section and may have a diameter that matches or is slightly larger than the diameter of a trocar cannula. This is to allow for a trocar cannula to slide in the longitudinal direction. For example, the diameter of the channel may be in the range of from 4 to 15 mm, e.g. from 5 to 12 mm.

In the first, open configuration, the diameter, d2, of the portion of the channel where the spring wire is arranged may correspond to the diameter of the remaining portions of the channel in the open configuration.

The diameter, d2, of the portion of the channel 104 in the first configuration may be at least 5%, e.g. between 5 and 10% larger than the diameter, d1, of the same portion of the channel 104 in the second configuration.

In the second configuration of the fixation device, the cross-sectional area of at least one portion of the channel where the spring wire is arranged may be from 5 to 20%, e.g. from 10-15% smaller than the cross-sectional area of that portion of the channel in the first configuration

As illustrated in e.g. figures 2A and 2B, the medical dressing 102 comprises a centrally disposed aperture 119 configured to encircle the annular member 103.

The diameter of the aperture 119 generally corresponds to the perimeter of the annular member 103. Accordingly, no gaps are formed between the fixation device 101 and the medical dressing 102. This is desirable to prevent the entry of contaminating microorganisms.

The annular member 103 may, in embodiments, be connected to or form part of a skin-attachment member 120.

The skin-attachment member 120 may, in embodiments, comprise a second annular member 121. The skin-attachment member 120 may form part of the fixation device 101. The (first) annular member 103 may be configured to mate with the second annular member 121 of the skin-attachment member 120. The first annular member 103 may be attached to the second annular member 121 by any means, e.g. by means of an adhesive or by means of welding.

The skin-attachment member 120 may further comprise a base member 122 arranged in contact with the skin of a patient. Alternatively, the annular member 103, which may also be referred to as a first annular member, is arranged in contact with the skin.

The second annular member 121 may comprise walls extending from a bottom surface to a top surface. The base member 122 may surround the bottom surface of the second annular member 121. The base member 122 may be configured to extend radially around the bottom surface of the annular member 103. The base member 122 has a larger surface area than the surface area of the second annular member 121.

The base member 122 may be formed integrally with the second annular member 121. The base member 122 is not limited to a particular shape, but is in figure 2B, circular.

In use, at least a portion of the medical dressing may overlie the base member 122 of the skin attachment member 120.

As illustrated in figures 2A and 2B, the medical dressing 102 is arranged between the base member 122 and annular member 103. The dressing may be clamped between the skin attachment member 120; i.e. the base member 122, and the annular member 103.

The medical dressing 102 is illustrated as a circular dressing in the figures. However, the medical dressing is by no means limited to a specific shape, but any shape is conceivable. It is also conceivable that the surgical staff may cut the dressing into a shape specifically adapted for a specific surgical intervention in an actual surgical scenario.

As best illustrated in figure 2B, the medical dressing 102 extends uninterrupted around the annular member 103.

Accordingly, the medical dressing 102 forms a closed path around the first annular member 104. Consequently, no part of the dressing is openable after the dressing has been applied to the skin. This is beneficial to secure a tight seal to the skin and also to prevent contaminants from entering the incision or the area of the skin circumventing the incision.

The medical dressing 102 may be detachably or fixedly attached to the fixation device 101. In embodiments, where the fixation device 101 comprises a skin-attachment member 120, the medical dressing 102 may be detachably or fixedly attached to the skin attachment member 120. For example, the medical dressing 102 may be detachably or fixedly attached to the base member 122 of the skin attachment member 120.

The detachable configuration of the medical dressing 102 allows for each of the components of the trocar fixation assembly to be packed separately such that the surgical staff may assemble the components of the fixation assembly prior to surgery.

In the fixed configuration, the trocar fixation assembly may be packaged as one individual component, which may save time since no assembly step is required prior to surgery.

As illustrated in the zoomed in, cross-sectional view in figure 2A, the medical dressing comprises at least a backing layer 110 and an adhesive skin contact layer 111.

The backing layer 110 is an outermost layer of the medical dressing and may also be referred to as a top layer. The backing layer 110 faces away from the patient's body in use.

The backing layer 110 may comprise a polymeric film, e.g. a polyethylene film, a polypropylene film or a polyurethane film. Preferably, the backing layer 110 comprises a polyurethane film. The thickness of the backing layer may be in the range of from 10 to 50 µm, e.g. from 15 to 40 µm.

As used herein, the term "adhesive skin contact layer" means a layer configured to detachably adhere the dressing to a dermal surface. In other words, the adhesive skin contact layer is configured to contact the skin of a wearer.

A release liner may being co-extensive with the adhesive skin contact layer 111 is typically detachably attached to the adhesive skin contact layer 111. The release liner prevents contamination of the adhesive skin contact layer 111 and is removed prior to application of the dressing to the skin.

The adhesive skin contact layer may comprise any skin-compatible adhesive.

Preferably, the adhesive skin contact layer 111 comprises a silicone based adhesive.

A silicone-based adhesive is gentle to the skin and may be removed from the skin without causing any trauma. For example, the adhesive skin contact layer 111 may comprise a silicone gel. The silicone gel may be provided as a coating on the backing layer or a pad, if present.

The adhesive skin contact layer 111 may comprise one or more sub-layers. For example, the adhesive skin contact layer 111 may comprise a polymeric film and an adhesive silicone gel layer, wherein the adhesive silicone gel layer is arranged to contact the skin.

The medical dressing may comprise an absorbent pad 112 between the backing layer 110 and the adhesive skin contact layer 111.

The absorbent pad 112 keeps the area circumventing the incision and the trocar fixation assembly free from blood. The absorbent pad 112 absorbs the blood and prevents maceration at the surgical site. Furthermore, the absorbent pad prevents contaminating microorganisms from accumulating at the skin or incision site.

In embodiments where the dressing comprises a pad, the backing layer 110 may be adhesively attached to the pad. Alternatively, the backing layer 110 may be laminated to the pad. For example, heat lamination may be utilized to apply the backing layer 110 to the pad.

The absorbent pad 112 preferably comprises a polyurethane foam.

Typically, the polyurethane foam is a hydrophilic polyurethane foam.

A polyurethane foam has a pressure relieving effect and is capable of absorbing large amounts of fluids

The pad 110 may comprise one or more layers. If the pad comprises a plurality of pad-forming layers, the pad-forming layers may be laminated or attached to each other to facilitate cutting through the dressing.

According to another aspect, there is provided a trocar system comprising a trocar fixation assembly 100 as described hereinbefore and a trocar 200.

The trocar system may be packaged and distributed in a unit such that the surgical staff does not need to assemble the trocar system prior to surgery.

Alternatively, the trocar fixation assembly, and the trocar, respectively, are packaged separately and assembled prior to use.

As illustrated in e.g. figure 5A, the trocar 200 comprises a cannula 201. The cannula serves as the "working channel" through which a variety of surgical tools can be inserted. The present disclosure is not limited to a particular cannula, but any cannula may be utilized.

The cannula is typically a hollow tube that serves as channel for the insertion of surgical tool. The cannula may be made of plastic or metal. The cannula may comprise a gas-tight valve 203 that provides for an internal air-seal allowing instruments to move in and out of the cannula without loss of pneumoperitoneum; i.e. gas present in the body cavity. The valve may be manually or automatically retractable during instrument passage.

The cannula 201 typically comprises an interior surface and an exterior surface 202. The exterior surface 202 of the cannula 201 may comprise ridges or threads or may be smooth. In the context of the present disclosure, a cannula 201 having a smooth exterior surface 202 is generally preferred.

The tip 204 of the cannula may be conical, blunt, eccentric or pyramidal to facilitate penetration and entry into a body cavity.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A trocar fixation assembly (100) comprising a fixation device (101) and a medical dressing (102), wherein said fixation device (101) comprises an annular member (103) defining a channel (104) for receiving a trocar; said annular member (103) being disposed about a longitudinal center line (105), wherein said fixation device (101) is configured to be movable between a first configuration, which allows for a trocar to be moved along said longitudinal center line (105) of said annular member (103), and a second configuration, which allows for a trocar to be fixed in a locked position, wherein in said second configuration, the cross-sectional area of at least one portion of said channel (104) is smaller than the cross-sectional area of said at least one portion of said channel (104) in said first configuration, wherein said fixation device (101) comprises locking means configured to move said fixation device (101) between said first and second configurations; said locking means comprising a spring member, wherein
said annular member (103) comprises interior walls (108a) and exterior walls (108b), and **characterized in that**
said spring member is a spring wire (106) encircling at least a portion of the interior walls (108a) of said annular member (103), and wherein said annular member (103) extends uninterrupted around said channel (104).

2. The trocar fixation assembly (100) according to claim 1, wherein said second configuration is a relaxed configuration and wherein said first configuration is a retracted configuration.

3. The trocar fixation assembly (100) according to claim 1 or claim 2, wherein said spring wire (106) is operably engaged with at least a first locking tab (107a), wherein said first locking tab (107a) projects radially from said exterior walls (108b) of said annular member (103).

4. The trocar fixation assembly (100) according to claim 3, wherein said first locking tab (107a) is movable between a first position and a second position, wherein the movement of said first locking tab (107a) from said first to said second position causes said spring wire (106) to retract such that said fixation device (101) is moved from said second to said first configuration.

5. The trocar fixation assembly (100) according to claim 3 or claim 4, wherein a distal end (116) of said spring wire (106) is connected to or forms part of said first locking tab (107a).

6. The trocar fixation assembly (100) according to any one of claims 3-5, wherein said annular member (103) comprises a second locking tab (107b) projecting radially from the exterior walls (108b) of said annular member (103), wherein said first locking tab (107a) is moveable in a circumferential direction with respect to said second locking tab (107b).

7. The trocar fixation assembly (100) according to any one of the preceding claims, wherein said medical dressing (102) comprises a centrally disposed aperture (119) configured to encircle said annular member (103).

8. The trocar fixation assembly (100) according to any one of the preceding claims, wherein said medical dressing (102) extends uninterrupted around said annular member (103).

9. The trocar fixation assembly (100) according to any one of the preceding claims, wherein said medical dressing comprises a backing layer (110) and an adhesive skin contact layer (111).

10. The trocar fixation assembly (100) according to claim 9, wherein said adhesive skin contact layer (111) comprises a silicone based adhesive.

11. The trocar fixation assembly (100) according to claim 9 or claim 10, wherein said medical dressing (102) comprises an absorbent pad (112) between said backing layer (110) and said adhesive skin contact layer (111).

12. The trocar fixation assembly (100) according to claim 11, wherein said absorbent pad (112) comprises a polyurethane foam.

13. A trocar system comprising a trocar fixation assembly (100) according to any one of the preceding claims and a trocar (200).

14. A trocar system according to claim 13, wherein said trocar (200) comprises a cannula (201), wherein said cannula (201) comprises an interior surface and an exterior surface (202), wherein said exterior surface (202) of said cannula (201) is smooth.

## Patentansprüche

1. Trokarfixierungsanordnung (100), die eine Fixierungsvorrichtung (101) und einen medizinischen Verband (102) umfasst, wobei die Fixierungsvorrichtung (101) ein ringförmiges Element (103) umfasst, das einen Kanal (104) zum Aufnehmen eines Trokars definiert; wobei das ringförmige Element (103) um eine Mittellinie in Längsrichtung (105) angeordnet ist, wobei die Fixierungsvorrichtung (101) so ausgelegt ist, dass sie zwischen einer ersten Gestaltung, die eine Bewegung des Trokars entlang der Mittellinie in Längsrichtung (105) des ringförmigen Elements (103) ermöglicht, und einer zweiten Gestaltung bewegbar ist, die eine Fixierung eines Trokars in einer verriegelten Stellung ermöglicht, wobei die Querschnittsfläche von mindestens einem Abschnitt des Kanals (104) in der zweiten Gestaltung kleiner ist als die Querschnittsfläche des mindestens einen Abschnitts des Kanals (104) in der ersten Gestaltung, wobei die Fixierungsvorrichtung (101) Verriegelungsmittel umfasst, die so ausgelegt sind, dass sie die Fixierungsvorrichtung (101) zwischen der ersten und zweiten Gestaltung bewegen; wobei die Verriegelungsmittel ein Federelement umfassen, wobei das ringförmige Element (103) Innenwände (108a) und Außenwände (108b) umfasst, und **dadurch gekennzeichnet, dass** das Federelement ein Federdraht (106) ist, der zumindest einen Abschnitt der Innenwände (108a) des ringförmigen Elements (103) umschließt, und wobei das ringförmige Element (103) ununterbrochen um den Kanal (104) verläuft.

2. Trokarfixierungsanordnung (100) nach Anspruch 1, wobei die zweite Gestaltung eine entspannte Gestaltung ist und wobei die erste Gestaltung eine zurückgezogene Gestaltung ist.

3. Trokarfixierungsanordnung (100) nach Anspruch 1 oder Anspruch 2, wobei der Federdraht (106) funktionsmäßig mit mindestens einem ersten Verriegelungsansatz (107a) verbunden ist, wobei der erste Verriegelungsansatz (107a) radial aus den Außenwänden (108b) des ringförmigen Elements (103) ragt.

4. Trokarfixierungsanordnung (100) nach Anspruch 3, wobei der erste Verriegelungsansatz (107a) zwischen einer ersten Stellung und einer zweiten Stellung bewegbar ist, wobei die Bewegung des ersten Verriegelungsansatzes (107a) aus der ersten in die zweite Stellung bewirkt, dass sich der Federdraht (106) zurückzieht, sodass die Fixierungsvorrichtung (101) aus der zweiten in die erste Gestaltung bewegt wird.

5. Trokarfixierungsanordnung (100) nach Anspruch 3 oder Anspruch 4, wobei ein distales Ende (116) des Federdrahts (106) mit dem ersten Verriegelungsansatz (107a) verbunden ist oder einen Bestandteil davon bildet.

6. Trokarfixierungsanordnung (100) nach einem der Ansprüche 3 bis 5, wobei das ringförmige Element (103) einen zweiten Verriegelungsansatz (107b) umfasst, der radial aus den Außenwänden (108b) des ringförmigen Elements (103) ragt, wobei der erste Verriegelungsansatz (107a) bezogen auf den zweiten Verriegelungsansatz (107b) in einer Umfangsrichtung bewegbar ist.

7. Trokarfixierungsanordnung (100) nach einem der vorhergehenden Ansprüche, wobei der medizinische Verband (102) eine mittig angeordnete Öffnung (119) umfasst, die so ausgelegt ist, dass sie das ringförmige Element (103) umschließt.

8. Trokarfixierungsanordnung (100) nach einem der vorhergehenden Ansprüche, wobei der medizinische Verband (102) ununterbrochen um das ringförmige Element (103) herum verläuft.

9. Trokarfixierungsanordnung (100) nach einem der vorhergehenden Ansprüche, wobei der medizinische Verband eine Trägerschicht (110) und eine haftende Hautkontaktschicht (111) umfasst.

10. Trokarfixierungsanordnung (100) nach Anspruch 9, wobei die haftende Hautkontaktschicht (111) einen Klebstoff auf Silikonbasis umfasst.

11. Trokarfixierungsanordnung (100) nach Anspruch 9 oder Anspruch 10, wobei der medizinische Verband (102) ein absorbierendes Kissen (112) zwischen der Trägerschicht (110) und der haftenden Hautkontaktschicht (111) umfasst.

12. Trokarfixierungsanordnung (100) nach Anspruch 11, wobei das absorbierende Kissen (112) einen Polyurethanschaum umfasst.

13. Trokarsystem, das eine Trokarfixierungsanordnung (100) nach einem der vorhergehenden Ansprüche und ein Trokar (200) umfasst.

14. Trokarsystem nach Anspruch 13, wobei der Trokar (200) eine Kanüle (201) umfasst, wobei die Kanüle (201) eine Innenfläche und eine Außenfläche (202) umfasst, wobei die Außenfläche (202) der Kanüle (201) glatt ist.

## Revendications

1. Ensemble de fixation de trocart (100) comprenant un dispositif de fixation (101) et un pansement médical (102), ledit dispositif de fixation (101) comprenant un élément annulaire (103) définissant un canal (104) destiné à recevoir un trocart ; ledit élément annulaire (103) étant disposé autour d'une ligne centrale longitudinale (105), ledit dispositif de fixation (101) étant configuré pour être mobile entre une première configuration, qui permet à un premier trocart d'être déplacé le long de ladite ligne centrale longitudinale (105) dudit élément annulaire (103), et une seconde configuration, qui permet à un trocart d'être fixé dans une position verrouillée, dans lequel, dans ladite seconde configuration, la superficie de section transversale d'au moins une section dudit canal (104) est inférieure à la superficie de section transversale de ladite au moins une section dudit canal (104) dans ladite première configuration, ledit dispositif de fixation (101) comprenant un moyen de verrouillage configuré pour déplacer ledit dispositif de fixation (101) entre lesdites première et seconde configurations ; ledit moyen de verrouillage comprenant un élément à ressort, ledit élément annulaire (103) comprenant des parois intérieures (108a) et des parois extérieures (108b) et étant **caractérisé en ce que** ledit élément à ressort est un fil à ressort (106) encerclant au moins une section des parois intérieures (108a) dudit élément annulaire (103), et ledit élément annulaire (103) s'étendant sans interruption autour dudit canal (104) .

2. Ensemble de fixation de trocart (100) selon la revendication 1, dans lequel ladite seconde configuration est une configuration déployée et ladite première configuration est une position rétractée.

3. Ensemble de fixation de trocart (100) selon la revendication 1 ou la revendication 2, dans lequel ledit fil à ressort (106) est engagé fonctionnellement dans une première languette de verrouillage (107a), ladite première languette de verrouillage (107a) saillant radialement depuis lesdites parois extérieures (108b) dudit élément annulaire (103).

4. Ensemble de fixation de trocart (100) selon la revendication 3, dans lequel ladite première languette de verrouillage (107a) est mobile entre une première position et une seconde position, le mouvement de ladite première languette de verrouillage (107a) de ladite première et ladite seconde position amenant ledit fil à ressort (106) à se rétracter de manière à ce que ledit dispositif de fixation (101) soit passé de ladite seconde à ladite première configuration.

5. Ensemble de fixation de trocart (100) selon la revendication 3 ou la revendication 4, dans lequel une extrémité distale (116) dudit fil à ressort (106) est connecté à ou fait partie intégrante de ladite première languette de verrouillage (107a).

6. Ensemble de fixation de trocart (100) selon l'une quelconque des revendications 3 à 5, dans lequel ledit élément annulaire (103) comprend une seconde languette de verrouillage (107b) saillant radialement depuis les parois extérieures (108b) dudit élément annulaire (103), ladite première languette de verrouillage (107a) étant mobile dans un sens circonférentiel par rapport à ladite seconde languette de verrouillage (107b).

7. Ensemble de fixation de trocart (100) selon l'une quelconque des revendications précédentes, dans lequel ledit pansement médical (102) comprend une ouverture pratiquée au centre (119) et configurée pour encercler ledit élément annulaire (103).

8. Ensemble de fixation de trocart (100) selon l'une quelconque des revendications précédentes, dans lequel ledit pansement médical (102) s'étend sans interruption autour dudit élément annulaire (103).

9. Ensemble de fixation de trocart (100) selon l'une quelconque des revendications précédentes, dans lequel ledit pansement médical comprend une couche support (110) et une couche adhésive en contact avec la peau (111) .

10. Ensemble de fixation de trocart (100) selon la revendication 9, dans lequel ladite couche adhésive en contact avec la peau (111) comprend un adhésif à base de silicone.

11. Ensemble de fixation de trocart (100) selon la revendication 9 ou 10, dans lequel ledit pansement médical (102) comprend un tampon absorbant (112) entre ladite couche support (110) et ladite couche adhésive en contact avec la peau (111).

12. Ensemble de fixation de trocart (100) selon la revendication 11, dans lequel ledit tampon absorbant (112) comprend une mousse de polyuréthane.

13. Système de trocart comprenant un ensemble de fixation de trocart (100) selon l'une quelconque des revendications précédentes et un trocart (200).

14. Système de trocart selon la revendication 13, ledit trocart (200) comprenant une canule (201), ladite canule (201) comprenant une surface intérieure et une surface extérieure (202), ladite surface extérieure (202) de ladite canule (201) étant lisse.
